# EUROPEAN PATENT APPLICATION

(11) **EP 0 701 812 A1**
(43) Date of publication of application: **20.03.1996**
(21) Application number: 95306345.0
(22) Date of filing: 11.09.1995
(51) Int. Cl.: A61K 7/32

(54) **Adsorption of sweat components with a macroporous copolymer**

(30) Priority: 16.09.1994 US 307121
(71) Applicant: DOW CORNING CORPORATION, Midland, Michigan 48686-0994 (US)
(72) Inventor: Abrutyn, Eric Steven, Midland, Michigan (US); Froix, Michael, Mountainview, California (US); Disapio, Alfred Joseph, Greenwich, Connecticut (US); Springer, Jeffery T., La Honda, California (US)
(74) Representative: Vandamme, Luc Johan Roger

(57) **Abstract**

A method of preventing underarm malodor associated with human perspiration. There is applied to the skin of a human, a safe and effective amount of a macroporous crosslinked copolymer containing acrylate or methacrylate units. Components of sweat such as fatty acids found in human perspiration, are adsorbed and entrapped into and within the confines of the macroporous copolymer. The macroporous copolymer possesses a pore diameter of a size sufficiently small to inhibit skin bacteria from entering. Decomposition of fatty acid and sweat components by the skin bacteria is thereby retarded, and hence the generation of body malodor is reduced. The macroporous copolymer may be applied to the human skin in the form of a deodorant stick, a deodorant cream, a deodorant lotion, a deodorant spray, a deodorant roll-on, a deodorant pad, a pressed powder deodorant, a loose powder deodorant or a deodorant gel.

## Description

This invention is directed to the adsorption of fatty acids and other components of sweat typically found in human perspiration. More particularly, it is directed to the adsorption and entrapment of sweat components within the confines of a macroporous copolymer. The macroporous copolymer must have a pore diameter sufficiently small to inhibit skin bacteria from entering.

Fatty acids such as propionic acid, butyric acid, and hexanoic acid, together with other components of sweat such as certain proteinaceous wastes, are among the primary components of axillary perspiration associated with body malodor. Upon reaching the surface of human skin, these materials are attacked by the bacterial flora which is pervasive on the skin. The materials are decomposed by the flora into volatile low molecular weight fatty acids such as isovaleric acid, caproic acid, and caprylic acid, in addition to certain mercaptans and aromatic amines, and hydrogen sulfide. These volatile products are the cause of body malodor.

Body malodor is most commonly combated with the aid of an underarm product such as a deodorant. Deodorants contain, among other ingredients, an antimicrobial agent which is intended to function in reducing the number of microorganisms found on the skin surface. Thus, the antimicrobial agent acts to reduce the bacterial flora present on the skin. In theory, this tends to reduce the decomposition which occurs on the skin, which in turn removes the cause of body malodor.

One of the most common antimicrobial agents employed as the active ingredient in underarm deodorant applications is TRICLOSAN. TRICLOSAN is the International Nomenclature Cosmetic Ingredient (INCI) name assigned by The Cosmetic, Toiletry and Fragrance Association (CTFA), Washington, D.C, for a substituted diphenyl ether compound, 5-chloro-2-(2,4-dichlorophenoxy) phenol or C₁₂H₇Cl₃O₂.

While TRICLOSAN is effective in its function in underarm deodorant applications, it as well as other commonly used antimicrobial agents are potentially "bad actors", in contributing to minor skin irritations in certain sensitive individuals. Therefore, a reduction in the amount of antimicrobial agent necessary for any given underarm application, would offer a distinct advantage in the marketplace, since a perceivable increase in mildness and a lower potential for skin irritation could result.

We have unexpectedly found that certain adsorbent copolymers exhibit effective sequestering of at least three fatty acids typically found in perspiration. While these copolymers are capable of entrapping the fatty acids and other sweat components because of their macroporous structure, they nevertheless possess a small enough pore diameter to inhibit entry of bacteria. By adsorbing fatty acids into the macroporous copolymer, these fatty acids are effectively isolated from decomposure by the bacterial flora found on skin, and the generation of body malodor is thereby significantly reduced.

Thus, the problem solved by the present invention involves the removal and isolation of fatty acids and other sweat components responsible for body malodor from skin flora to reduce the generation of body odor. According to the invention, this problem is solved by selectively adsorbing and entrapping the fatty acids and other sweat components within the confines of a highly crosslinked macroporous copolymer.

Thus, our invention introduces a method of preventing underarm malodor associated with human perspiration. According to the invention, there is applied to the skin of a human, a safe and effective amount of a macroporous crosslinked copolymer. Components of sweat, in particular fatty acids found in human perspiration, are surprisingly adsorbed and entrapped within the confines of said copolymer. The macroporous copolymer possesses a pore diameter of a size sufficiently small to inhibit skin bacteria from entering. Decomposition of these fatty acids by the skin bacteria is thereby retarded, and hence the generation of body malodor is reduced.

Preferably, the macroporous copolymer has pores with a distribution of pore diameters of between 0.0001 to 0.1 micrometer. The macroporous copolymer is in the form of particles having a particle size of between 1 to 80 micrometers in diameter. The macroporous copolymer may be applied to the skin as an ingredient of a formulated underarm product such as a deodorant stick, a deodorant cream, a deodorant lotion, a deodorant spray, a deodorant roll-on, a deodorant pad, a deodorant pressed powder, a deodorant loose powder or a deodorant gel.

Adsorbent macroporous copolymers are described in U.S. Patent 4690825, US Patent 4724240 and in a published European Patent Application 0 369 741 A2. Such materials are sold under the trademarks MICROSPONGE® by Advanced Polymer Systems Inc., Redwood City, California, and as POLYTRAP® by Dow Corning Corporation Midland, Michigan.

Evaluations of these adsorbent copolymers have demonstrated their effective sequestration of perspiration fatty acids such as propionic acid, butyric acid, and hexanoic acid, from solutions of these acids which approximate normal concentrations of human perspiration. These adsorbent copolymers inhibit the ingress of skin bacteria but allow the ingress of the fatty acids. In underarm applications, these adsorbent copolymers exhibit positive sensory characteristics.

By including such copolymers as an ingredient of a deodorant product, it is possible to minimize or prevent the bacterial decomposition of perspiration components responsible for body malodor. Simultaneously, the deodorant products are imbued with improved sensory aesthetics. These copolymers have not been found to be incompatible with other deodorant ingredients such as fragrances and substantivity aids, regardless of form delivered to the skin.

The perspiration fatty acids are preferably "entrapped" within particles of a macroporous highly crosslinked copolymer containing acrylate or methacrylate units. These particles are not water soluble. Such macroporous materials are preferably manufactured in the form of spherical beads. Other forms of the adsorbent material can be employed such as a complex POLYTRAP® particulate which consists of a mixture of unit particles, agglomerates and aggregates.

These hydrophobic copolymeric materials are used to entrap the fatty acids and other components of sweat and are highly macroporous. Thus, the materials possess an inordinate amount of interstitial space including a vast labyrinth of voids. The material is capable of adsorbing several times its own weight of sweat components including fatty acids. Since the process involved is adsorption in contrast to absorption, the properties of both the copolymeric material and the entrapped sweat components are not altered.

The fatty acids are entrapped within the material in contrast to being encapsulated. Encapsulation connotes a complete enclosing of one material within another such as a shell formed around a core of liquid. Encapsulated ingredients are released by mechanical disruption of the shell or dissolution of the shell. Once the shell is disrupted, the entire contents of the shell are released or extracted. In entrapment, however, the release of an entrapped component is controlled or sustained by wicking, evaporation or capillary action. Mechanical disruption is not required.

The discrete particles of the hydrophobic macroporous materials of the present invention are free flowing particulates, even when loaded with an entrapped component. One copolymer representative of one species of a genus of these materials has the formula:

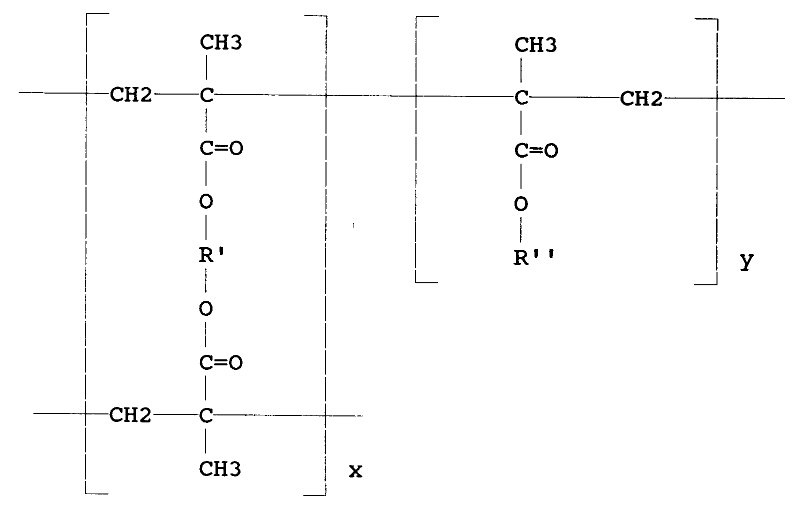

In this species of copolymer, x and y are integers in which the ratio of x:y is from 1:99 to 99:1. R' is the alkylene radical (-CH2CH2-)a, in which a is an integer having a value of from one to eight. R'' is the alkyl group -(CH2)bCH3 in which b has a value of from zero to twenty-nine. In a more limited form of this particular species, the ratio of x to y is about 80:20. R' is -CH2CH2- and R'' is -CH₃ or -(CH2)11CH3, although the genus of these copolymeric materials is not so limited.

Suspension polymerization is one preferred technique which is used to produce these adsorbent macroporous materials. According to that particular technique, polymerization is carried out in water. The monomers, a volatile hydrocarbon solvent or material acting as a porogenic agent and a catalyst are combined and form beads or droplets in water and polymerization occurs within each bead. A surfactant or a stabilizer such as polyvinyl pyrrolidone is required to prevent the individually formed beads and droplets from coalescing in the solution. The resulting beads have a substantially spherical outer crust or shell, but reveal an interior macroporous structure.

The bead is typically 1 to 80 micrometers in average diameter. The particle diameter is controlled as a function of the rate of agitation used during the suspension polymerization process. The macroporous copolymer must have a pore diameter, in this application, of a size sufficiently small to inhibit skin bacteria from entering. Thus, a pore diameter of between 0.0001 to 0.1 micrometer is most preferred. The process for making the macroporous copolymer with such a controlled pore diameter is described in detail in US Patent 4690825. For example, according to that patent, the porosity is increased by increasing the crosslinking density or by increasing the concentration of the porogenic agent in the solution.

Other stabilizers and protective colloids such as starch, polyvinyl alcohol, carboxymethyl cellulose, methyl cellulose, or inorganic divalent alkali metal hydroxides such as MgOH, may also be used in place of polyvinyl pyrrolidone in this suspension polymerization process. When the volatile hydrocarbon solvent or porogenic agent is removed, it leaves behind an empty porous copolymer bead.

Typically, two or more monomers are used in the process. One monomer is usually a polyunsaturated monomer and the other monomer is a monounsaturated monomer. Representative polyunsaturated monomers which are used are ethylene glycol dimethacrylate, triethylene glycol dimethacrylate, tetraethylene glycol dimethacrylate; 1,3 butyleneglycol dimethacrylate; 1,4 butanediol dimethacrylate; 1,6 hexanediol dimethacrylate, neopentyl glycol dimethacrylate, bisphenol A dimethacrylate; divinylbenzene and trivinylbenzene; divinyltoluene and trivinyltoluene; triallyl maleate, triallyl phosphate, diallyl maleate and diallyl itaconate.

The monounsaturated monomers which are used are preferably methacrylates and acrylates having straight-chain, branched-chain, or closed rings, with 5 to 30 carbon atoms, and more particularly those containing 5 to 18 carbon atoms. Representative of such preferred monounsaturated monomers are methyl methacrylate, lauryl methacrylate, 2-ethylhexyl methacrylate, methylhexyl methacrylate, isodecyl methacrylate, stearyl methacrylate, cyclohexyl methacrylate, and dimethylamino ethyl methacrylate. Other types of monounsaturated monomers such as styrene can also be used, however.

Highly crosslinked copolymeric systems consisting of particles of submicrometer size can also be prepared from monomers having at least two polymerizable unsaturated bonds and with no comonomers having a monounsaturated moiety. The details of the processes for making these adsorbent copolymers are found in any one of three patents, namely US Patent 4690825, US Patent 4724240 or the published European Patent Application 0 369 741 A2.

Standard texts generally define a fatty acid as a carboxylic acid derived from, or contained in an animal or vegetable fat or oil, and composed of a chain of alkyl groups of from 4 to 22 carbon atoms characterized by a terminal carboxyl group -COOH. However, for purposes of this invention, a "fatty acid" is also intended to include acids such as acetic acid and propionic acid.

The invention is illustrated in more detail in the following example. In the example, three of the known aliphatic carboxylic acid components of human sweat are used to study the sequestering power of macroporous copolymers sold under the trademark MICROSPONGE®. All of the macroporous MICROSPONGE® copolymers tested exhibited the ability to immobilize the three components of sweat. The three acids were propionic acid, butyric acid and hexanoic acid. These acids, however, were not equally sequestered by the macroporous copolymer adsorbent. It was found that the higher molecular weight aliphatic acid was retained the most.

Propionic acid, butyric acid, and hexanoic acid, were dissolved in water. A gas chromatographic assay was utilized with the aid of a polar NUKOL™ megabore Supelco™ column in a Hewlett-Packard™ 5890 chromatograph with split injection. Standard curves of the three acids were generated. To test each sample for sequestration, 100 milligrams of MICROSPONGE® macroporous copolymer was mixed with 10 milliliters of 500 parts per million of sweat acid. The MICROSPONGE® employed in these tests had a pore diameter which was between 0.0001 to 0.1 micrometer and a particle diameter which was between 1 to 80 micrometers. The solution was agitated on a rotating table for one hour, filtered through a 0.45 micrometer filter, and then injected into the gas chromatograph. The retention times for the three acids were 1.8 minutes for propionic acid, 2.4 minutes for butyric acid, and 4.2 minutes for hexanoic acid. The filtrate was analyzed after removal of the MICROSPONGE® macroporous copolymer. The concentration of the starting solution was 546 parts per million for propionic acid, 573 parts per million for butyric acid, and 533 parts per million for hexanoic acid. It was found that the acid remaining in the filtrate was about 75 percent for propionic acid, about 75 percent for butyric acid, and about 25 percent for hexanoic acid. Correspondingly, the amount of acid sequestered by the macroporous copolymer was about 25 percent for propionic acid, about 25 percent for butyric acid and about 75 percent for hexanoic acid.

The macroporous copolymer can be applied to the human skin in the form of a deodorant stick, a deodorant cream, a deodorant lotion, a deodorant spray, a deodorant roll-on, a deodorant pad, a deodorant pressed powder, a deodorant loose powder or a deodorant gel. Formulations for delivery of the macroporous copolymer in such deodorant applications are known in the art.

Thus, for delivery of the macroporous copolymer as a deodorant aerosol, there is combined with the macroporous copolymer, a propellant such as butane, isobutane, propane, isopropane, nitrogen, or carbon dioxide; a suspending agent such as an organofunctional clay, ethanol, or propylene carbonate; an aesthetic enhancer such as CYCLOMETHICONE, DIMETHICONE, isopropyl myristate, isopropyl palmitate, or a C₁₂ to C₁₅ alcohol benzoate; and a sensory additive such as a fragrance or talc.

For delivery of the macroporous copolymer as a deodorant roll-on, there is combined with the macroporous copolymer, an aesthetic enhancer such as CYCLOMETHICONE, an organic ester, or dioctyl adipate; a sensory additive such as a fragrance, talc, silica, or polyethylene; a solubilizer such as water or an alcohol; a viscosity builder such as silica, aluminum starch, or octenyl succinate; and an emulsifier such as glycerol monostearate, STEARETH 2, an alkoxylate, or an organofunctional clay.

For delivery of the macroporous copolymer as a deodorant solid such as a stick, there is combined with the macroporous copolymer, a solidifier such as sodium stearate, cetyl alcohol, or stearyl alcohol; an aesthetic enhancer such glycerin, propylene glycol, CYCLOMETHICONE, a C₁₂ to C₁₅ alcohol benzoate, an organic ester or an organic ether; and a sensory additive such as an emulsifier, a fragrance, talc, silica or polyethylene.

Other deodorant forms known in the art which are suitable for delivery of the macroporous copolymer are creams, pads, lotions, gels or powders. These other forms contain similar ingredients as those noted above. When added to a deodorant product, the product should contain 0.5 to 5 percent by weight of the macroporous copolymer. Amounts in excess of five percent can also be employed, to attain adequate adsorption of the fatty acids and other of the sweat components if necessary.

The hydrophobic macroporous copolymer is utilized in combination with a volatile component such as CYCLOMETHICONE, a fragrance, or ethanol, which is first incorporated into the copolymer and then into a deodorant product. Upon application to the axilla, the volatile component is volatilized, leaving the macroporous copolymer free to adsorb and entrap the components of sweat into and within confines of the copolymer.

## Claims

1. A method of preventing underarm malodor associated with human perspiration comprising applying to the skin of a human, a safe and effective amount of a macroporous crosslinked copolymer containing acrylate or methacrylate units; sequestering and isolating the components of sweat found in human perspiration from decomposure by skin bacteria by adsorbing and entrapping the components of sweat found in human perspiration within the confines of the macroporous copolymer; the macroporous copolymer having a pore diameter of a size sufficiently small so as to inhibit skin bacteria from entering; whereby the components of sweat found in human perspiration are removed and isolated from decomposition by the skin bacteria and the generation of body malodor is reduced.

2. A method according to claim 1 in which the sweat components are fatty acids selected from the group consisting of butyric acid, propionic acid and hexanoic acid.

3. A method according to claim 1 in which the macroporous copolymer has a pore diameter of between 0.0001 to 0.1 micrometer.

4. A method according to claim 3 in which the macroporous copolymer is in the form of particles having a particle size of between 1 to 80 micrometers in diameter.

5. A method according to claim 2 in which there is adsorbed and entrapped within the confines of the macroporous copolymer an amount of from 25 to 75 percent by weight of the fatty acids.

6. A method according to claim 1 in which the macroporous copolymer is applied to the skin in a form selected from the group consisting of a deodorant stick, a deodorant cream, a deodorant lotion, a deodorant spray, a deodorant roll-on, a deodorant pad, a deodorant pressed powder, a deodorant loose powder and a deodorant gel.
